(19) Europäisches Patentamt
European Patent Office
Office européen des brevets

(11) Veröffentlichungsnummer : **0 343 462 B1**

(12) # EUROPÄISCHE PATENTSCHRIFT

(45) Veröffentlichungstag der Patentschrift : **11.12.91 Patentblatt 91/50**

(51) Int. Cl.$^5$ : **C07D 239/42,** C07D 251/16, C07D 251/46, C07D 403/12, C07D 409/12, A01N 47/44

(21) Anmeldenummer : **89108652.2**

(22) Anmeldetag : **13.05.89**

(54) **Aminoguanidinoazine.**

Die Akte enthält technische Angaben, die nach dem Eingang der Anmeldung eingereicht wurden und die nicht in dieser Patentschrift enthalten sind.

(30) Priorität : **27.05.88 DE 3818040**

(43) Veröffentlichungstag der Anmeldung : **29.11.89 Patentblatt 89/48**

(45) Bekanntmachung des Hinweises auf die Patenterteilung : **11.12.91 Patentblatt 91/50**

(84) Benannte Vertragsstaaten : **BE CH DE ES FR GB IT LI NL SE**

(56) Entgegenhaltungen :
**EP-A- 0 121 082**
**EP-A- 0 173 319**
**EP-A- 0 173 320**
**EP-A- 0 224 078**

(73) Patentinhaber : **BAYER AG**
**W-5090 Leverkusen 1 Bayerwerk (DE)**

(72) Erfinder : **Fest, Christa, Dr.**
**Im Johannistal 20**
**W-5600 Wuppertal 1 (DE)**
Erfinder : **Kirsten, Rolf, Dr.**
**Carl-Langhans-Strasse 27**
**W-4019 Monheim (DE)**
Erfinder : **Kluth, Joachim, Dr.**
**Kurt-Schumacher-Strasse 9**
**W-4018 Langenfeld (DE)**
Erfinder : **Müller, Klaus Helmut, Dr.**
**Bockhackstrasse 55**
**W-4000 Düsseldorf 13 (DE)**
Erfinder : **Riebel, Hans Jochem, Dr.**
**In der Beek 92**
**W-5600 Wuppertal 1 (DE)**
Erfinder : **Santel, Hans-Joachim, Dr.**
**Gruenstrasse 9 a**
**W-5090 Leverkusen 1 (DE)**
Erfinder : **Schmidt, Robert R., Dr.**
**Im Waldwinkel 110**
**W-5060 Bergisch Gladbach 2 (DE)**
Erfinder : **Strang, Harry, Dr.**
**Unterdorfstrasse 6 a**
**W-4000 Düsseldorf 31 (DE)**

**EP 0 343 462 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue Aminoguanidinoazine, ein Verfahren zu ihrer Herstellung und ihre Verwendung als Herbizide.

Es ist bereits bekannt, daß bestimmte substituierte Aminoguanidinoazine, wie z. B. N'-(4,6-Dimethyl-pyrimidin-2-yl)-N''-acetylamino-N'''-(2-chlor-phenylsulfonyl)-guanidin, herbizide Eingenschaften aufweisen (vgl. EP-A 121082). Die herbizide Wirkung der bisher bekannten Aminoguanidinoazine ist jedoch nicht immer ganz zufriedenstellend.

Es wurden nun neue Aminoguanidinoazine der allgemeinen Formel (I)

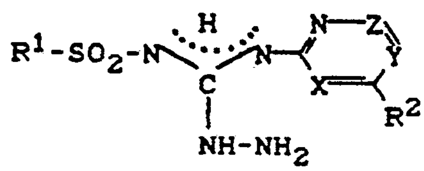

(I)

in welcher

$R^1$ für den Rest 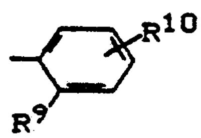 steht worin

$R^9$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxycarbonyl steht und

$R^{10}$ für Wasserstoff oder Chlor steht ; worin weiter

$R^1$ für den Rest 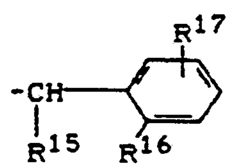 steht, worin

$R^{15}$ für Wasserstoff steht

$R^{16}$ für Fluor, Chlor, Brom, Methyl, Methoxy, Ethoxy, Difluormethoxy, Trifluormethoxy, Methoxycarbonyl, Ethoxycarbonyl, Methylsulfonyl oder Dimethylaminosulfonyl steht und

$R^{17}$ für Wasserstoff oder Chlor steht ; worin weiter

$R^1$ für den Rest 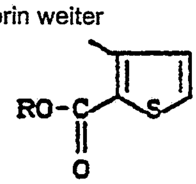 steht, worin

R für $C_1$-$C_2$-Alkyl steht, oder

$R^1$ für den Rest 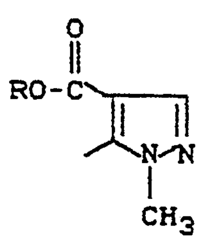 steht, worin

R für $C_1$-$C_2$-Alkyl steht ;

in welcher weiter

$R^2$ für Wasserstoff, Fluor, Chlor, Brom, Methyl, Methoxymethyl, Dimethoxymethyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff steht,

Y für Stickstoff oder eine —$CR^3$-Gruppierung steht, worin

$R^3$ für Wasserstoff, Fluor, Chlor oder Methyl steht, und

Z für eine —$CR^4$-Gruppierung steht, worin

$R^4$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht,

sowie Salze von Verbindungen der Formel (I) gefunden, wobei folgende Verbindungen ausgenommen sind : N'-(4,6-Dimethyl-pyrimidin-2-yl)-N''-amino-N'''-(2-diethylaminosulfonyl-phenylsulfonyl)-guanidin (vgl. US-P 4725303, Beisp. 230) ; N'-(4,6-Dimethyl-pyrimidin-2-yl)-N''-amino-N'''-(2-methoxycarbonyl-phenylsulfonyl)-guanidin (vgl. US-P 4725303, Beisp. 201) und N'-(4,6-Dimethyl-pyrimidin-2-yl)-N''-amino-N'''-(2-methoxycarbonyl-thiophen-3-yl-sulfonyl)-guanidin (vgl. EP-A 224078, Beisp. 27).

Die allgemeine Formel (I) steht für die einzelnen möglichen Tautomeren der Formeln (IA), (IB) und (IC)

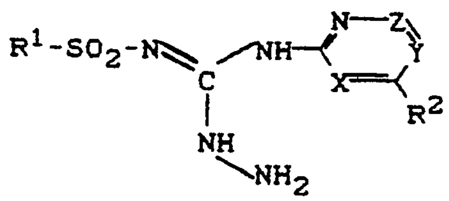

(IA)

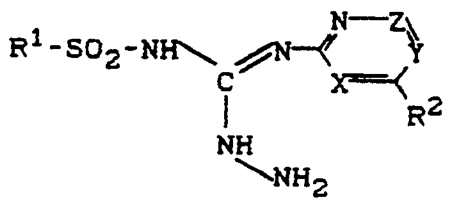

(IB)

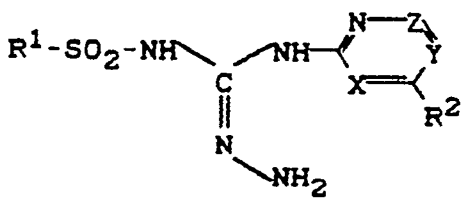

(IC)

sowie für Gemische dieser Tautomeren.

Man erhält die neuen Aminoguanidine der allgemeinen Formel (I) wenn man Sulfonylverbindungen der allgemeinen Formel (II)

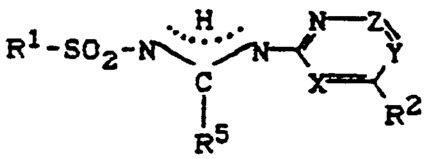

(II)

in welcher

| | |
|---|---|
| $R^1$, $R^2$, X, Y und Z | die oben angegebenen Bedeutungen haben und |
| $R^5$ | für Halogen vorzugweise für Chlor oder eine der nachstehend angegebenen Abgangsgruppen |

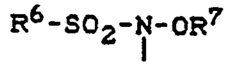

oder —Q-$R^8$ steht, worin

- $R^6$ die oben für $R^1$ angegebene Bedeutung hat, aber nicht in jedem Einzelfall mit $R^1$ identisch sein muß,
- $R^7$ für $C_1$-$C_4$-Alkyl, $C_3$-$C_4$-Alkenyl oder Benzyl steht,
- $R^8$ für gegebenenfalls durch Carboxy, $C_1$-$C_4$-Alkoxy-carbonyl oder $C_1$-$C_4$-Alkoxy substituiertes $C_1$-$C_4$-Alkyl oder für jeweils gegebenenfalls durch Fluor, Chlor, Brom, $C_1$-$C_4$-Alkyl oder $C_1$-$C_4$-Alkoxy substituiertes Benzyl oder Phenyl steht, und
- Q für Sauerttoff oder Schwefel steht,

mit Hydrazin oder einem Hydrazin-Wasser- oder Hydrazin-Säuro-Addukt, gegebenenfalls in Gegenwart eines Säureakzeptors und gegebenentalls in Gegenwart eines Verdünnungsmittels umsetzt und gegebenenfalls die so erhaltenen Produkte nach üblichen Methoden in Salze überführt.

Die neuen Aminoguanidinoazine der allgemeinen Formel (I) zeichnen sich durch starke herbizide Wirksamkeit aus.

Überraschenderweise zeigen die neuen Verbindungen der allgemeinen Formel (I) erheblich bessere herbizide Wirkung als vorbekannte Aminoguanidinoazine gleicher Wirkungsrichtung.

Die Erfindung betrifft weiter vorzugsweise Salze von Verbindungen der Formel (I)

- α) mit Protonensäuren, wie z. B. Salzsäure, Schwefelsäure, Phosphorsäure, Methansulfonsäure, Benzol- oder p-Toluolsulfonsäure, oder Naphthalin-mono- oder -di-sulfonsäuren, oder
- β) mit Basen, wie z. B. Natrium-, Kalium- oder Calcium-hydroxid, -hydrid, -amid oder carbonat, Natrium- oder Kalium-$C_1$-$C_4$-alkanolaten, Ammoniak, $C_1$-$C_4$-Alkylaminen, Di-($C_1$-$C_4$-alkyl)-aminen oder Tri-($C_1$-$C_4$-alkyl)-aminen.

Verwendet man N'-(4,6-Dimethoxy-s-triazin-2-yl)-N''-methoxy-N'',N'''-bis-(2-brom-phenylsulfonyl)-guanidin und Hydrazin als Ausgangsstoffe, so kann der Reaktions- ablauf beim erfindungsgemäßen Verfahren durch folgendes Formelschema skizziert werden :

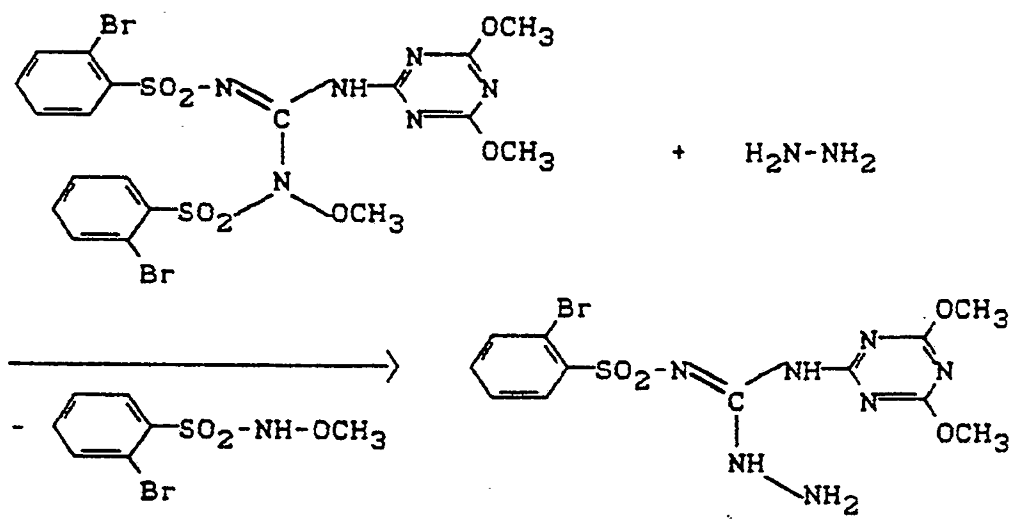

Die beim erfindungsgemäßen Verfahren als Ausgangsstoffe zu verwendenden Sulfonylverbindungen sind durch die Formel (II) allgemein definiert.

Beispiele für die Ausgangsstoffe der Formel (II) sind in der nachstehenden Tabelle I aufgeführt.

Tabelle I : Beispiele für die Ausgangsstoffe der Formel (II)

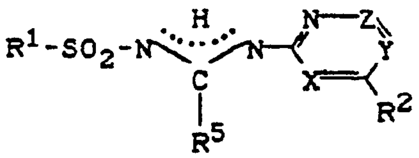

(II)

**Tabelle 1**

| $R^5$ | $R^1$ | $R^2$ | X | Y | Z |
|---|---|---|---|---|---|
| 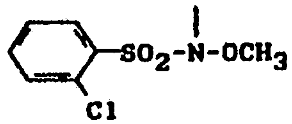 | 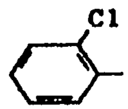 | $CH_3$ | N | CH | $C-OCH_3$ |
| 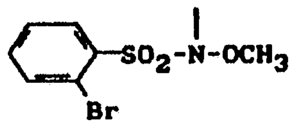 | 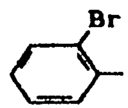 | $OCH_3$ | N | CH | $C-OCH_3$ |
| 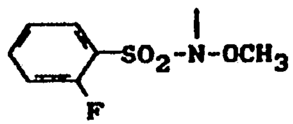 | 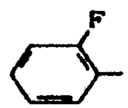 | $OCH_3$ | N | CH | $C-OCH_3$ |
| 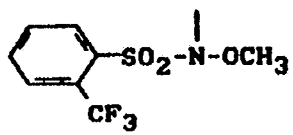 | 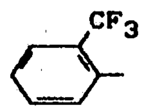 | $OCH_3$ | N | CH | $C-OCH_3$ |
| 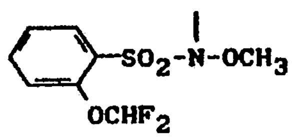 | 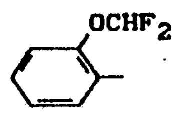 | $OCH_3$ | N | CH | $C-OCH_3$ |

**Tabelle 1** - Fortsetzung

| $R^5$ | $R^1$ | $R^2$ | X | Y | Z |
|---|---|---|---|---|---|
| 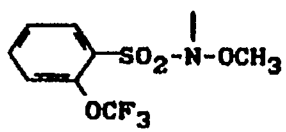 | 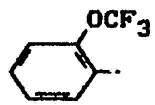 | $OCH_3$ | N | CH | $C-OCH_3$ |
| 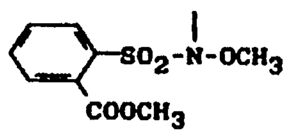 | 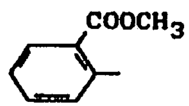 | $CH_3$ | N | CH | $C-CH_3$ |
| 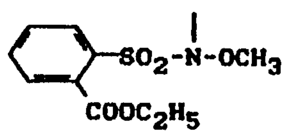 | 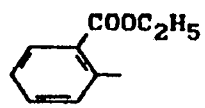 | $CH_3$ | N | CH | $C-CH_3$ |
| 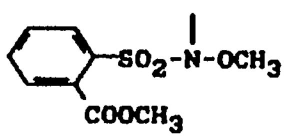 | 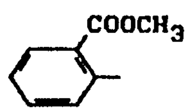 | $OCH_3$ | N | CH | $C-OCH_3$ |
| 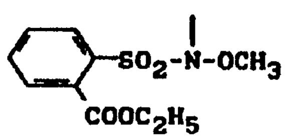 | 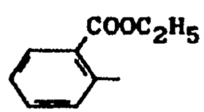 | $OCH_3$ | N | CH | $C-OCH_3$ |

**Tabelle 1** - Fortsetzung

| $R^5$ | $R^1$ | $R^2$ | X | Y | Z |
|---|---|---|---|---|---|
| 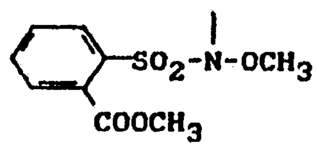 | 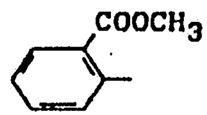 | C-$OCH_3$ | N | CH | $C_2H_5$ |
| 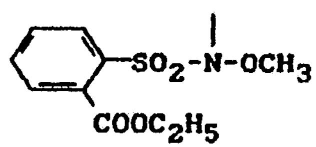 | 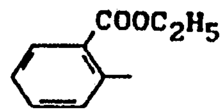 | $OCH_3$ | N | CH | C-$C_2H_5$ |
| 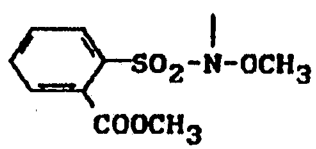 | 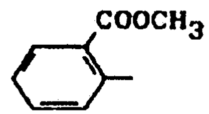 | $OCH_3$ | N | CH | C-Cl |
| 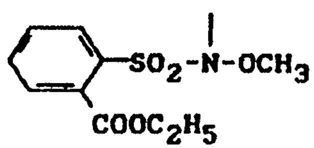 | 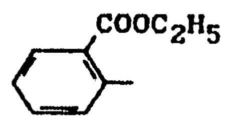 | $OCH_3$ | N | CH | C-Cl |
| 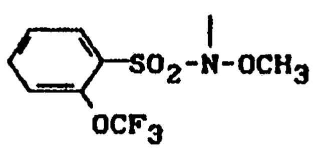 | 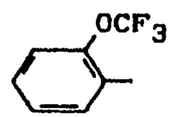 | $OCH_3$ | N | CH | C-Cl |

**Tabelle 1** - Fortsetzung

| $R^5$ | $R^1$ | $R^2$ | X | Y | Z |
|---|---|---|---|---|---|
| 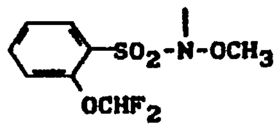 | 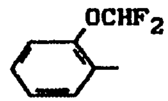 | $OCH_3$ | N | CH | C-Cl |
| 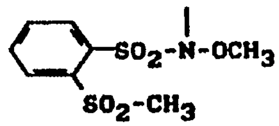 | 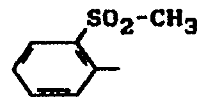 | H | N | CH | $C-CH_3$ |
| 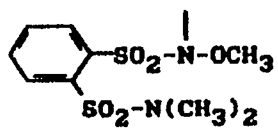 | 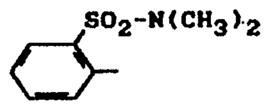 | $CH_3$ | N | CH | $C-OCH_3$ |
| 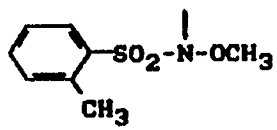 | 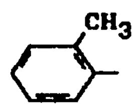 | $OCH_3$ | N | CH | $C-OCH_3$ |
| 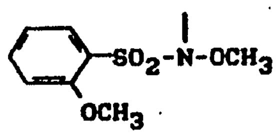 | 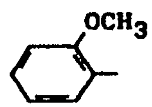 | $CH_3$ | N | CH | $C-OCH_3$ |

**Tabelle 1** - Fortsetzung

| $R^5$ | $R^1$ | $R^2$ | X | Y | Z |
|---|---|---|---|---|---|
| 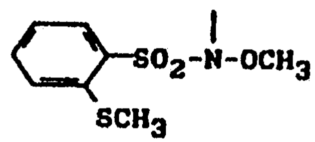 | 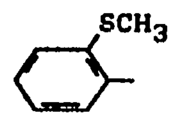 | $OCH_3$ | N | CH | $C-OCH_3$ |
| 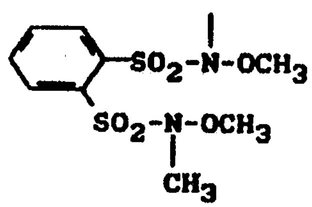 | 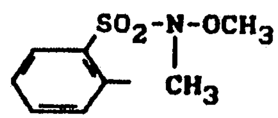 | $OCH_3$ | N | CH | $C-OCH_3$ |
| 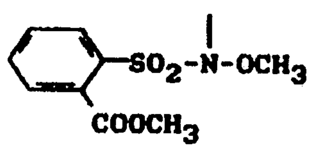 | 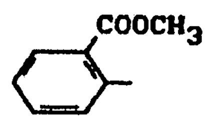 | $CH_3$ | N | CH | $C-OC_2H_5$ |
| 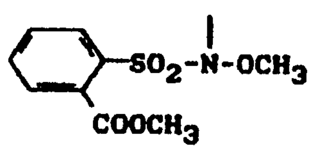 | 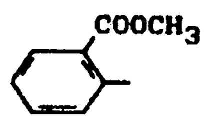 | $OCHF_2$ | N | CH | $C-CH_3$ |

**Tabelle 1** - Fortsetzung

| $R^5$ | $R^1$ | $R^2$ | X | Y | Z |
|---|---|---|---|---|---|
| 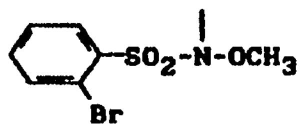 | 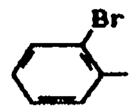 | $CH_3$ | N | CH | $C-SCH_3$ |
| 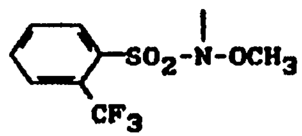 | 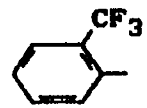 | $CH_3$ | N | CH | $C-N(CH_3)_2$ |
| 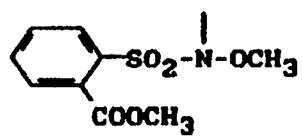 | 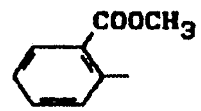 | $OCHF_2$ | N | CH | $C-OCHF_2$ |
| 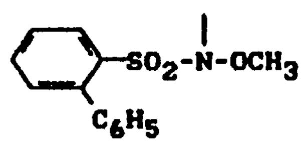 | 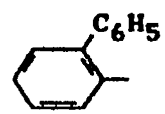 | $OCH_3$ | N | CH | $C-OCH_3$ |
| 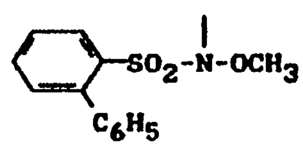 | 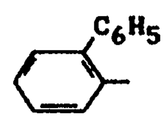 | $OCH_3$ | N | N | $C-OCH_3$ |

**Tabelle 1** - Fortsetzung

| $R^5$ | $R^1$ | $R^2$ | X | Y | Z |
|---|---|---|---|---|---|
| 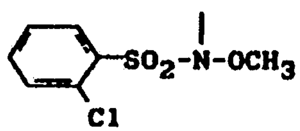 | 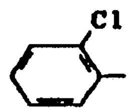 | $CH_3$ | N | N | $C-OCH_3$ |
| 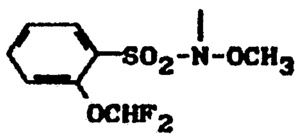 | 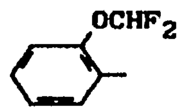 | $OCH_3$ | N | N | $C-OCH_3$ |
| 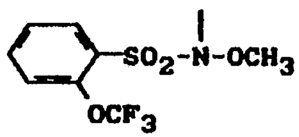 | 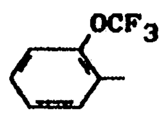 | $OCH_3$ | N | N | $C-OCH_3$ |
| 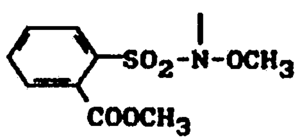 | 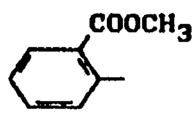 | $CH_3$ | N | N | $C-OCH_3$ |
| 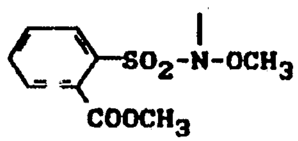 | 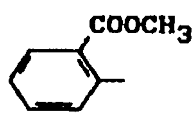 | $OCH_3$ | N | N | $C-OCH_3$ |

**Tabelle 1** - Fortsetzung

| $R^5$ | $R^1$ | $R^2$ | X | Y | Z |
|---|---|---|---|---|---|
| 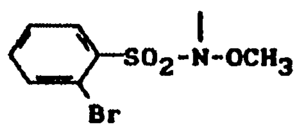 | 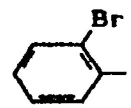 | $CH_3$ | N | N | $C-CH_3$ |
| 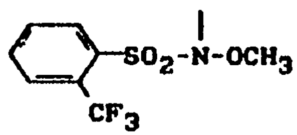 | 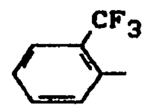 | $CH_3$ | N | N | C-Cl |
| 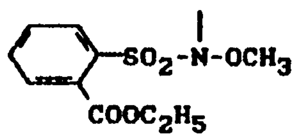 | 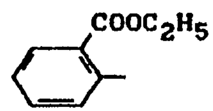 | $OCH_3$ | N | N | $C-OCH_3$ |
| 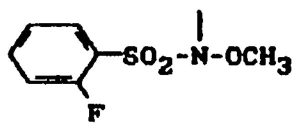 | 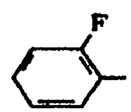 | $OCH_3$ | N | N | $C-OCH_3$ |
| 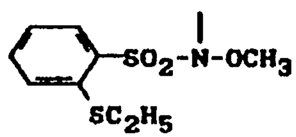 | 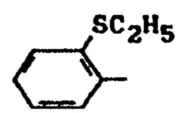 | $OCH_3$ | N | N | $C-OCH_3$ |

**Tabelle 1** - Fortsetzung

| $R^5$ | $R^1$ | $R^2$ | X | Y | Z |
|---|---|---|---|---|---|
| 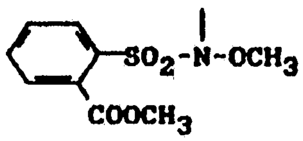 | 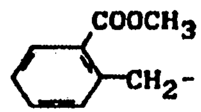 | $OCH_3$ | N | CH | $C-OCH_3$ |
| 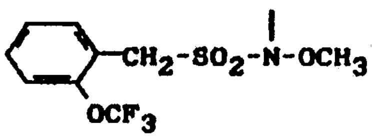 | 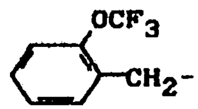 | $OCH_3$ | N | CH | $C-OCH_3$ |
| $-OC_6H_5$ | 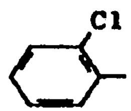 | $CH_3$ | N | CH | $C-CH_3$ |
| $-OCH_3$ | 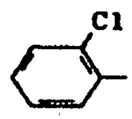 | $CH_3$ | N | CH | $C-OCH_3$ |
| $-SCH_3$ | 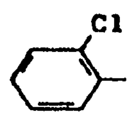 | $OCH_3$ | N | CH | $C-OCH_3$ |

**Tabelle 1** - Fortsetzung

| $R^5$ | $R^1$ | $R^2$ | X | Y | Z |
|---|---|---|---|---|---|
| $-SC_6H_5$ | 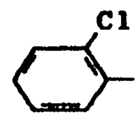 | $OCH_3$ | N | CH | $C-OCH_3$ |
| 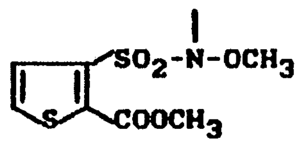 | 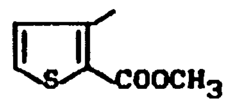 | $CH_3$ | N | N | $C-OCH_3$ |
| 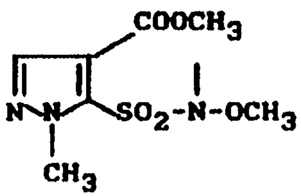 | 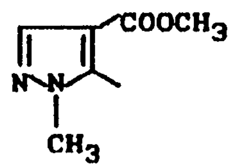 | $OCH_3$ | N | CH | $C-OCH_3$ |
| 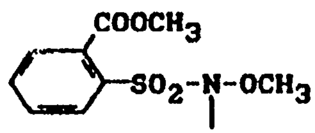 | 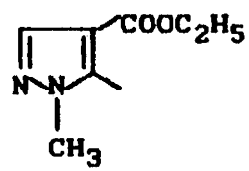 | $OCH_3$ | N | CH | $C-OCH_3$ |

**Tabelle 1** - Fortsetzung

| $R^5$ | $R^1$ | $R^2$ | X | Y | Z |
|---|---|---|---|---|---|
| Cl | 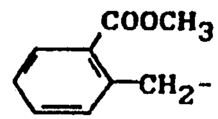 | $OCH_3$ | N | CH | $C-OCH_3$ |
| $-OC_6H_5$ | 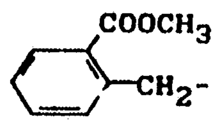 | $OCH_3$ | N | CH | $C-OCH_3$ |
| $-SC_6H_5$ | 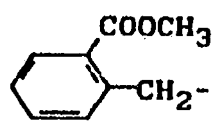 | $OCH_3$ | N | CH | $C-OCH_3$ |
| Cl | 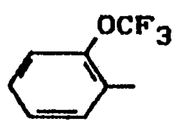 | $CH_3$ | N | N | $C-OCH_3$ |
| 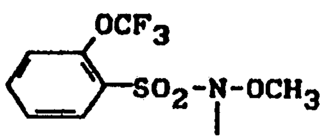 | 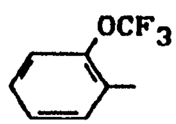 | $CH_3$ | N | N | $C-OCH_3$ |

<u>Tabelle 1</u> - Fortsetzung

| $R^5$ | $R^1$ | $R^2$ | X | Y | Z |
|---|---|---|---|---|---|
| $S-C_6H_5$ | 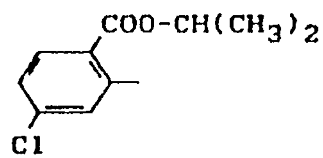 | $CH_3$ | N | N | $C-OCH_3$ |
| $S-CH_3$ | 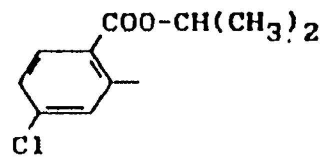 | $CH_3$ | N | N | $O-CH_3$ |

<u>Tabelle 1</u> - Fortsetzung

| $R^5$ | $R^1$ | $R^2$ | X | Y | Z |
| --- | --- | --- | --- | --- | --- |
| $S-CH_3$ | 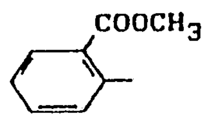 | $CH(OCH_3)_2$ | N | CH | $O-CH_3$ |
| $S-C_6H_5$ | 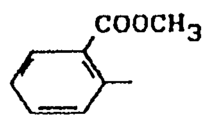 | $CH(OCH_3)_2$ | N | CH | $O-CH_3$ |
| $S-CH_3$ | 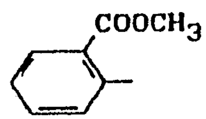 | $NH-CH_3$ | N | N | $O-C_2H_5$ |
| 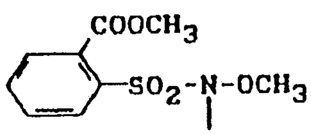 | 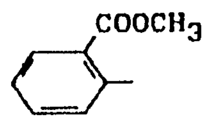 | $NH-CH_3$ | N | N | $O-C_2H_5$ |

Die Ausgangsstoffe der Formel (II) sind bekannt und/oder können nach an sich bekannten Verfahren hergestellt werden (vgl. EP-A 5986, EP-A 24215, EP-A 121082, EP-A 172957, EP-A 173321, EP-A 173956, EP-A 224078, DE-OS 3634928, DE-OS 3634929).

Das erfindungsgemäße Verfahren wird unter Einsatz von Hydrazin oder einem Hydrazin-Wasser- oder Hydrazin-Säure-Addukt durchgeführt.

Vorzugsweise kommen praktisch wasserfreies Hydrazin oder Hydrazin-Hydrat sowie Hydrazin-Addukte mit Mineralsäuren, wie z. B. Hydrazin-mono- oder -di-hydrochlorid und Hydrazinsulfat in Betracht.

Diese Verbindungen sind bekannte Synthesechemikalien.

Das erfindungsgemäße Verfahren zur Herstellung der neuen Aminoguanidinoazine der Formel (I) wird vorzugsweise unter Verwendung von Verdünnungsmitteln durchgeführt. Als Verdünnungsmittel kommen dabei vorzugsweise Wasser und/oder polare organische Lösungsmittel, wie Methanol, Ethanol, Isopropanol, Butanol, Isobutanol, sec-Butanol, tert-Butanol, Glycoldimethylether, Diglycoldimethylether, Tetrahydrofuran, Dioxan, Essigsäuremethylester, Essigsäureethylester, Acetonitril, Propionitril, Dimethylformamid, Dimethylacetamid, N-Methylpyrrolidon, Dimethylsulfoxid und Tetramethylensulfon, in Betracht.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen −20°C und +80°C, vorzugsweise bei Temperaturen zwischen +0°C und 50°C.

Das erfindungsgemäße Verfahren wird im allgemeinen bei Normaldruck durchgeführt.

Zur Durchführung des erfindungsgemäßen Verfahrens setzt man je Mol Sulfonylverbindung der Formel (II) im allgemeinen zwischen 1 und 3 Mol, vorzugsweise zwischen 1,0 und 1,5 Mol, Hydrazin bzw. Hydrazin-Wasser-Addukt bzw. Hydrazin-Säure-Addukt ein.

Im allgemeinen werden die Reaktionskomponenten bei Raumtemperatur oder unter Eiskühlung zusammen gegeben und das Reaktionsgemisch wird, gegebenenfalls bei erhöhter Temperatur, bis zum Ende der Umsetzung gerührt. Die Pro dunkte der Formel (I) fallen im allgemeinen nach dem Abkühlen kristallin an und können durch Absaugen isoliert werden.

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z. B. Wasser, Methanol, Ethanol oder Aceton, und Zugabe einer geeigneten Säure bzw. Base. Die Salze können dann — gegebenenfalls nach längerem Rühren — durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthi u m, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Zierge hölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage : z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln ; als Emulgier- und/oder schaumerzeugende Mittel kommen in Frage : z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit

bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4 (1H,3H)-dion (AMETHYDIONE) oder N-(2-Benzthiazolyl)-N,N'-dimethyl-harnstoff (METABENZTHIAZURON) zur Unkrautbekämpfung in Getreide ; 4-Amino-3-methyl-6-phenyl-1,2,4-triazin-5 (4H)-on (METAMITRON) zur Unkrautbekämpfung in Zuckerrüben und 4-Amino-6-(1,1-dimethylethyl)-3-methylthio-1,2,4-triazin-5 (4H)-on (METRIBUZIN) zur Unkrautbekämpfung in Sojabohnen, in Frage ; ferner auch 2,4-Dichlorphenoxyessigsäure (2,4-D) ; 4-(2,4-Dichlorphenoxy)-buttersäure (2,4-DB) ; 2,4-Dichlorphenoxypropionsäure (2,4-DP) ; 2,6-Dichlorbenzonitril (DICHLOBENIL) ; 4-Amino-6-t-butyl-3-ethylthio-1,2,4-triazin-5 (4H)-on (ETHIOZIN) ; N-Phosphonomethyl-glycin (GLYPHOSATE) ; 3-Cyclohexyl-6-dimethylamino-1-methyl-1,3,5-triazin-2,4-dion (HEXAZINONE) ; 2-(4,5-Dihydro-4-methyl-4-isopropyl-5-oxo-1H-imidazol-2-yl)-pyridin-3-carbonsäure (IMAZAPYR) ; (2-Methyl-4-chlorphenoxy)-essigsäure (MCPA) ; (4-Chlor-2-methylphenoxy)-propionsäure (MCPP) ; N-Methyl-2-(1,3-benzthiazol-2-yloxy)-acetanilid (MEFENACET) ; 2-Chlor-4,6-bis-(ethylamino)-1,3,5-triazin (SIMAZIN) und Methyl-2-{[(4,6-dimethyl-2-pyrimidinyl)-aminocarbonyl]-aminosulfonyl}-benzoat (SULFOMETURON) ; außerdem (insbesondere zur Anwendung in Getreide) :
2-[4-(2,4-Dichlorphenoxy)-phenoxy]-propionsäure, deren Methylester oder Ethylester (DICLOFOP),
2-{4-[(6-Chlor-2-benzoxazolyl)-oxyl]-phenoxy}-propansäure, deren Methylester oder Ethylester (FENOXAPROP),
N-(1-Ethylpropl)-3,4-dimethyl-2,6-dinitroanilin (PENDIMETHALIN),
Methyl-5-(2,4-dichlorphenoxy)-2-nitrobenzoat (BIFENOX),
N,N-Dimethyl-N'-(3-chlor-4-methylphenyl)-harnstoff (CHLORTOLURON),
N,N-Dimethyl-N'-(4-isopropylphenyl)-harnstoff (ISOPROTURON),
Methyl-2-[4,5-dihydro-4-methyl-4-(1-methylethyl)-5-oxo-1H-imidazol-2-yl]-4(5)-methylbenzoat (IMAZAMETHABENZ),
3,5-Dibrom-4-hydroxy-benzonitril (BROMOXYNIL),
3,5-Diiod-4-hydroxy-benzonitril (IOXYNIL),
2-Chlor-N-{[4-methoxy-6-methyl-1,3,5-triazin-2-yl)-amino]-carbonyl}benzolsulfonamid (CHLORSULFURON),
2-{[[((4-Methoxy-6-methyl-1,3,5-triazin-2-yl)-amino)-carbonyl]-amino]-sulfonyl}-benzoesäure oder deren Methylester (METSULFURON),
2{[(4,6-Dimethyl-pyrimidin-2-yl)-aminocarbonyl]-aminosulfonyl}-benzoesäure oder deren Methylester (SULFMETURON),
3-[[[[(4-Methoxy-6-methyl-1,3,5-triazin-2-yl)-amino]-carbonyl]-sulfonyl]-thiophen-2-carbonsäure-methylester (THIAMETURON),
2,3,3-Trichlorallyl-diisopropyl-thiocarbamat (TRIALLAT),
4-Ethylamino-2-t-butylamino-6-methylthio-s-triazin (TERBUTRYNE),
3-Isopropyl-2,1,3-benzothiadiazin-4-on-2,2-dioxid (BENTAZON),
O-(6-Chlor-3-phenyl-pyridazin-4-yl)-S-octyl-thiocarbonat (PYRIDATE).

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

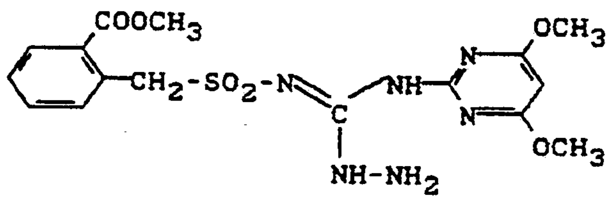

1,3 g (0,025 Mol) Hydrazinhydrat werden bei anfangs 20°C zu einer Suspension von 15,9 g (0,025 Mol) N'-(4,6-Dimethoxy-pyrimidin-2-yl)-N''-methoxy-N''-(2-methoxycarbonyl-phenylsulfonyl)-N'''-(2- methoxycarbonyl-benzylsulfonyl)-guanidin in 100 ml Methanol unter Rühren gegeben, wobei sich die Reaktionsmischung auf 30°C erwärmt und eine klare Lösung entsteht. Das nach vierstündigem Rühren bei 20°C bis 30°C kristallin abgeschiedene Produkt wird durch Absaugen isoliert.

Man erhält 9,5 g (89% der Theorie) N'-(4,6-Dimethoxy-pyrimidin-2-yl)-N''-amino-N'''-(2-methoxycarbonyl-benzylsulfonyl)-guanidin vom Schmelzpunkt 166°C.

Analog Beispiel 1 und entsprechend der allgemeinen Beschreibung des erfindungsgemäßen Verfahrens können die in der nachstehenden Tabelle 2 aufgeführten Verbindungen der Formel (I) hergestellt werden.

Tabelle 2 : Beispiele für die Verbindungen der Formel (I)

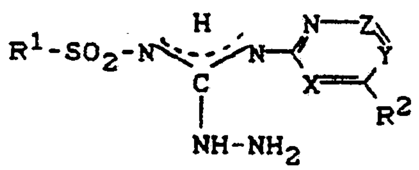

(I)

| Bsp.-Nr. | $R^1$ | $R^2$ | X | Y | Z | Schmelzpunkt (°C) |
|---|---|---|---|---|---|---|
| 2 | 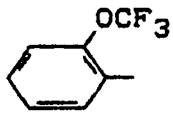 | $OCH_3$ | N | CH | $C-OCH_3$ | 134 |
| 3 | 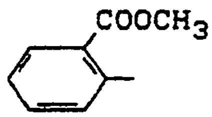 | $OCH_3$ | N | CH | $C-OCH_3$ | 159 |
| 4 | 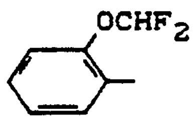 | $CH_3$ | N | CH | $C-CH_3$ | 98 |
| 5 | 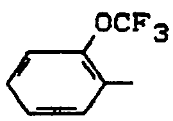 | $CH_3$ | N | N | $C-OCH_3$ | amorph |
| 6 | 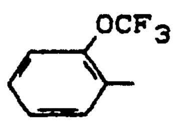 | $OCH_3$ | N | N | $C-OCH_3$ | amorph |
| 7 | 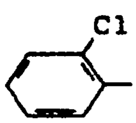 | $CH_3$ | N | CH | $C-OCH_3$ | 166 |
| 8 | 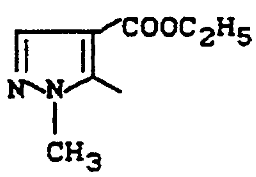 | $CH_3$ | N | CH | $C-CH_3$ | 105 |

Tabelle 2 - Fortsetzung

| Bsp.-Nr. | $R^1$ | $R^2$ | X | Y | Z | Schmelzpunkt (°C) |
|---|---|---|---|---|---|---|
| 9 | 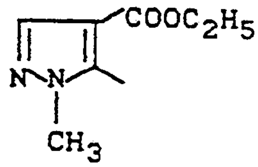 | $CH_3$ | N | N | $C-CH_3$ | 102 |
| 10 | 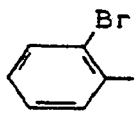 | $CH_3$ | N | CH | $C-CH_3$ | |
| 11 | 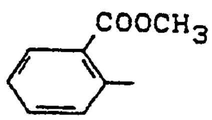 | $CH_3$ | N | N | $C-OCH_3$ | |
| 12 | 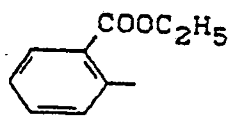 | $CH_3$ | N | CH | $C-OCH_3$ | |
| 13 | 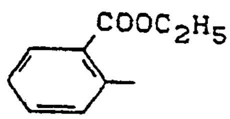 | $OCH_3$ | N | CH | $C-OCH_3$ | |
| 14 | 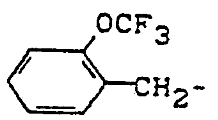 | $OCH_3$ | N | CH | $C-OCH_3$ | |
| 15 | 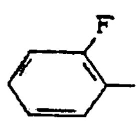 | $OCH_3$ | N | N | $C-OCH_3$ | |

Tabelle 2 - Fortsetzung

| Bsp.-Nr. | $R^1$ | $R^2$ | X | Y | Z | Schmelzpunkt (°C) |
|---|---|---|---|---|---|---|
| 16 | 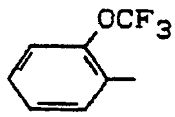 | $OCH_3$ | N | CH | $C-C_2H_5$ | |
| 17 | 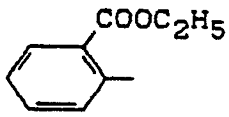 | $CH_3$ | N | N | $C-OCH_3$ | |
| 18 | 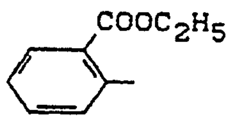 | $OCH_3$ | N | N | $C-OCH_3$ | |
| 19 | 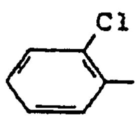 | $CH_3$ | N | N | $C-OCH_3$ | |
| 20 | 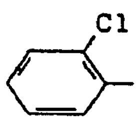 | $OCH_3$ | N | N | $C-OCH_3$ | 175 |
| 21 | 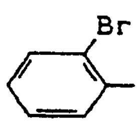 | $OCH_3$ | N | N | $C-OCH_3$ | |
| 22 | 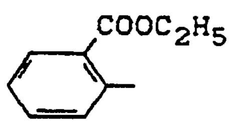 | $CH_3$ | N | CH | $C-CH_3$ | |
| 23 | 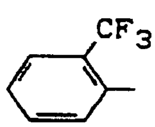 | $CH_3$ | N | CH | $C-CH_3$ | |

Tabelle 2 - Fortsetzung

| Bsp.-Nr. | $R^1$ | $R^2$ | X | Y | Z | Schmelzpunkt (° C) |
|---|---|---|---|---|---|---|
| 24 | 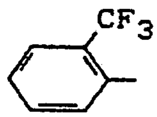 | $OCH_3$ | N | CH | C-$OCH_3$ | |
| 25 | 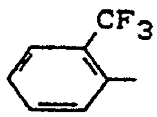 | $CH_3$ | N | N | C-$OCH_3$ | |
| 26 | 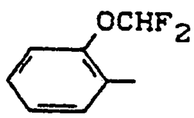 | $OCH_3$ | N | CH | C-$OCH_3$ | |
| 27 | 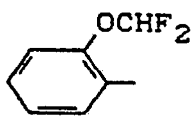 | $OCH_3$ | N | N | C-$OCH_3$ | |
| 28 | 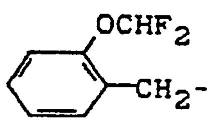 | $OCH_3$ | N | CH | C-$OCH_3$ | |
| 29 | 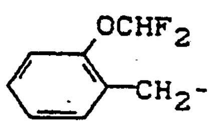 | $OCH_3$ | N | N | C-$OCH_3$ | |
| 30 | 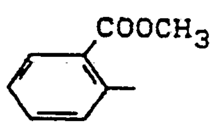 | $OCH_3$ | N | N | C-$C_2H_5$ | |
| 31 | 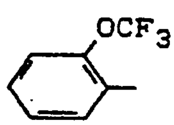 | $CH_3$ | N | CH | C-$OC_2H_5$ | |

Tabelle 2 - Fortsetzung

| Bsp.-Nr. | $R^1$ | $R^2$ | X | Y | Z | Schmelzpunkt (°C) |
|---|---|---|---|---|---|---|
| 32 | 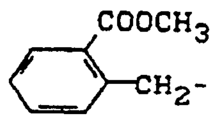 | $OCH_3$ | N | N | $C-OCH_3$ | |
| 33 | 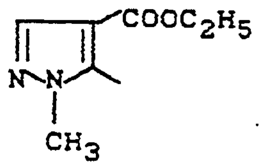 | $OCH_3$ | N | CH | $C-OCH_3$ | 125 |
| 34 | 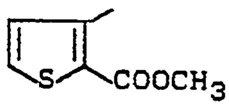 | $CH_3$ | N | N | $C-OCH_3$ | |
| 35 | 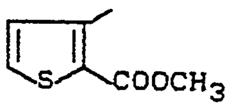 | $OCH_3$ | N | N | $C-OCH_3$ | |
| 36 | 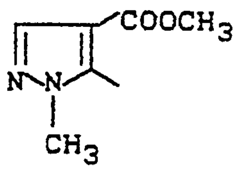 | $OCH_3$ | N | N | $C-OCH_3$ | |
| 37 | 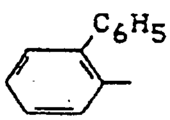 | $OCH_3$ | N | CH | $C-OCH_3$ | |
| 38 | 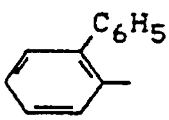 | $OCH_3$ | N | N | $C-OCH_3$ | 143 |
| 39 | 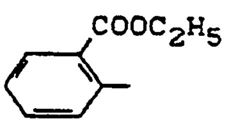 | $OCH_3$ | N | CH | C-Cl | |

Tabelle 2 - Fortsetzung

| Bsp.-Nr. | $R^1$ | $R^2$ | X | Y | Z | Schmelzpunkt (°C) |
|---|---|---|---|---|---|---|
| 40 | 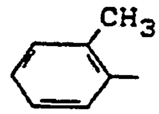 | $CH_3$ | N | N | $C-OCH_3$ | |
| 41 | 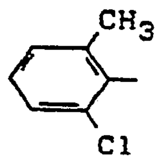 | $CH_3$ | N | CH | $C-CH_3$ | |
| 42 | 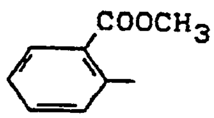 | $CF_3$ | N | CH | $C-OCH_3$ | |
| 43 | 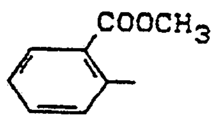 | $CH_3$ | N | CH | $C-OCHF_2$ | |
| 44 | 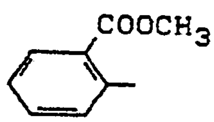 | $OCH_3$ | N | CH | $C-OCHF_2$ | |
| 45 | 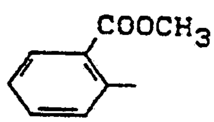 | $OCHF_2$ | N | CH | $C-OCHF_2$ | |
| 46 | 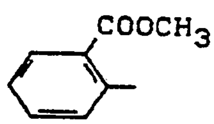 | $NHCH_3$ | N | N | $C-OC_2H_5$ | |
| 47 | 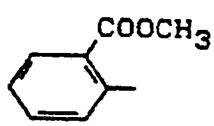 | $NHC_2H_5$ | N | N | $C-OCH_3$ | |

Tabelle 2 - Fortsetzung

| Bsp.-Nr. | $R^1$ | $R^2$ | X | Y | Z | Schmelzpunkt (°C) |
|---|---|---|---|---|---|---|
| 48 | 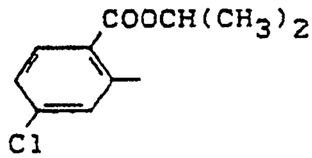 | $CH_3$ | N | N | $C-OCH_3$ | |
| 49 | 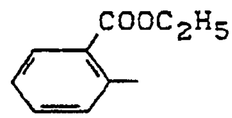 | Cl | N | CH | $C-OCH_3$ | |
| 50 | 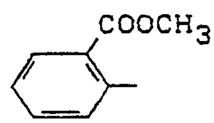 | $CH(OCH_3)_2$ | N | CH | $C-OCH_3$ | |
| 51 | 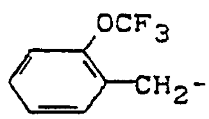 | $OCH_3$ | N | N | $C-OCH_3$ | |
| 52 | 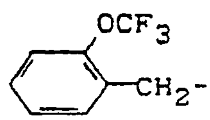 | $CH_3$ | N | N | $C-OCH_3$ | |
| 53 | 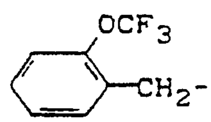 | $CH_3$ | N | N | $C-CH_3$ | |
| 54 | 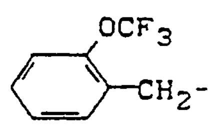 | $NHCH_3$ | N | N | $C-OC_2H_5$ | |
| 55 | 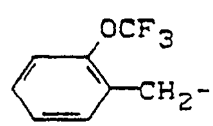 | $OCH_3$ | N | N | $C-C_2H_5$ | |

Tabelle 2 - Fortsetzung

| Bsp.-Nr. | $R^1$ | $R^2$ | X | Y | Z | Schmelzpunkt (°C) |
|---|---|---|---|---|---|---|
| 56 | 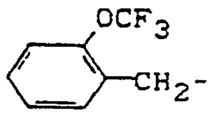 | $CH_3$ | N | N | $C-OC_2H_5$ | |
| 57 | 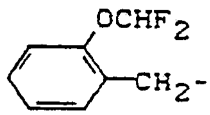 | $CH_3$ | N | N | $C-OCH_3$ | |
| 58 | 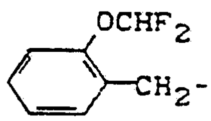 | $CH_3$ | N | N | $C-CH_3$ | |
| 59 | 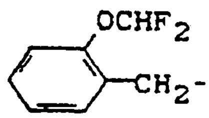 | $NHCH_3$ | N | N | $C-OC_2H_5$ | |
| 60 | 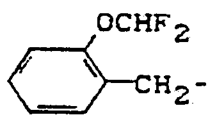 | $OCH_3$ | N | N | $C-C_2H_5$ | |
| 61 | 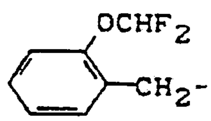 | $CH_3$ | N | N | $C-OC_2H_5$ | |
| 62 | 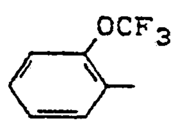 | $CF_3$ | N | CH | $C-OCH_3$ | |
| 63 | 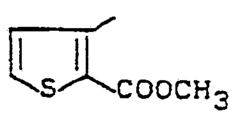 | $OCHF_2$ | N | CH | $C-OCHF_2$ | |
| 64 | 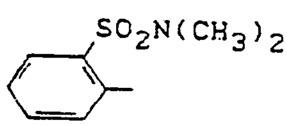 | $CH_3$ | N | CH | $C-OCH_3$ | |

Tabelle 2 - Fortsetzung

| Bsp.-Nr. | $R^1$ | $R^2$ | X | Y | Z | Schmelzpunkt (°C) |
|---|---|---|---|---|---|---|
| 65 | 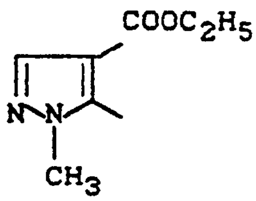 | $CH_3$ | N | CH | $C-OCH_3$ | 105 |
| 66 | 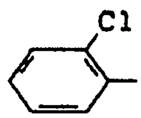 | $CH_3$ | N | CH | $C-CH_3$ | 139 |
| 67 | 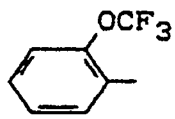 | $CH_3$ | N | N | $C-N(CH_3)_2$ | 246 |
| 68 | 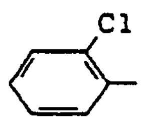 | $CH_3$ | N | N | $C-N(CH_3)_2$ | 163 |
| 69 | 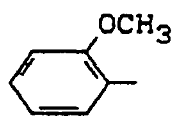 | $CH_3$ | N | N | $C-N(CH_3)_2$ | 132 |
| 70 | 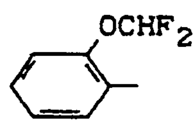 | $CH_3$ | N | N | $C-N(CH_3)_2$ | 197 |
| 71 | 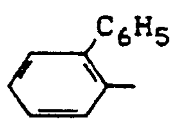 | $CH_3$ | N | N | $C-OCH_3$ | 162 |
| 72 | 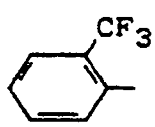 | $CH_3$ | N | N | $C-SCH_3$ | 216 |

Tabelle 2 - Fortsetzung

| Bsp.-Nr. | $R^1$ | $R^2$ | X | Y | Z | Schmelzpunkt (°C) |
|---|---|---|---|---|---|---|
| 73 | 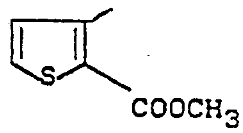 | $OCH_3$ | N | CH | $C-OCH_3$ | 163 |
| 74 | 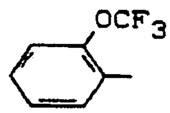 | $CH_3$ | N | CH | $C-CH_3$ | 60 |
| 75 | 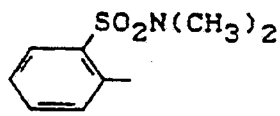 | $CH_3$ | N | CH | $C-CH_3$ | 172 |
| 76 | 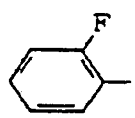 | $CH_3$ | N | CH | $C-CH_3$ | 159 |
| 77 | 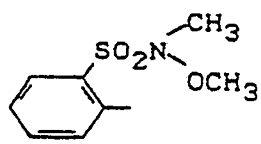 | $CH_3$ | N | CH | $C-CH_3$ | 197 |
| 78 | 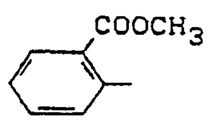 | H | N | CH | $C-CH_3$ | |
| 79 | 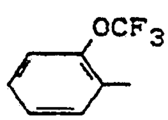 | H | N | CH | $C-CH_3$ | |
| 80 | 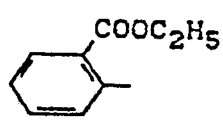 | H | N | CH | $C-CH_3$ | |
| 81 | 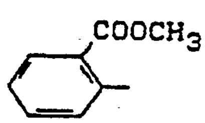 | $CH_2OCH_3$ | N | CH | $C-OCH_3$ | |

Herstellung der in Tabelle 2 als Beispiel 112 aufgeführten Verbindung :

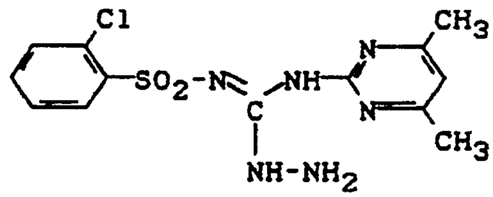

3,4 g (8,2 mMol) N'-(4,6-Dimethyl-pyrimidin-2-yl)-N''-(2-chlor-phenylsulfonyl)-O-phenyl-isoharnstoff werden in 50 ml Tetrahydrofuran gelöst und mit 0,4 g (8,2 mMol) Hydrazinhydrat versetzt. Das Reaktionsgemisch wird 3 Stunden bei 20°C bis 25°C gerührt, dann auf 250 ml Eiswasser gegossen und mit Methylenchlorid extrahiert. Die organische Phase wird mit Wasser gewaschen, mit Natriumsulfat getrocknet und filtriert. Vom Filtrat wird das Lösungsmittel im Wasserstrahlvakuum abdestilliert und der Rückstand wird aus Isopropanol kristallisiert. Man erhält 2,0 g (69% der Theorie) N'-(4,6-Dimethyl-pyrimidin-2-yl)-N''-amino-N'''-(2-chlor-phenylsulfonyl)-guanidin vom Schmelzpunkt 139°C.

Anwendungsbeispiele

In den folgenden Anwendungsbeispielen wird die nachstehend aufgeführte Verbindung als Vergleichssubstanz herangezogen :

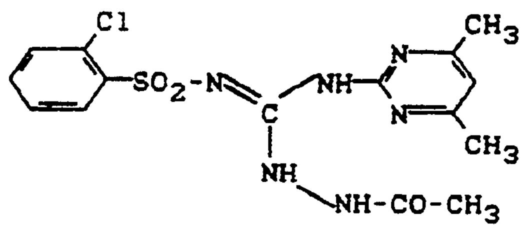

(A)

N'-(4,6-Dimethyl-pyrimidin-2-yl)-N''-acetylamino-N'''-(2-chlor-phenylsulfonyl)-guanidin (bekannt aus EP-A 121082).

Beispiel A

Pre-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0% = keine Wirkung (wie unbehandelte Kontrolle)
100% = totale Vernichtung

Eine deutliche Überlegenheit in der Wirksamkeit gegenüber dem Stand der Technik zeigen in diesem Test z. B. die Verbindungen gemäß Herstellungsbeispielen 2, 3, 4, 7, 64, 65, 66.

Beispiel B

Post-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrölle.

Es bedeuten :

0% = keine Wirkung (wie unbehandelte Kontrolle)
100% = totale Vernichtung

Eine deutliche Überlegenheit in der Wirksamkeit gegenüber dem Stand der Technik zeigen in diesem Test z.B, die Verbindungen gemäß Herstellungsbeispielen 1, 2, 3, 4, 5, 6, 7, 64, 65, 66.

## Patentansprüche

**Patentansprüche für folgende Vertragsstaaten : BE, CH, DE, FR, GB, IT, LI, NL, SE**

1. Aminoguanidinoazine der allgemeinen Formel (I)

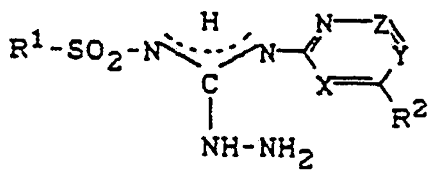

(I)

in welcher

$R^1$ für den Rest 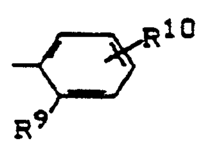 steht worin

$R^9$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phonoxy oder $C_1$-$C_3$-Alkoxycarbonyl steht und
$R^{10}$ für Wasserstoff oder Chlor steht ; worin weiter

$R^1$ für den Rest 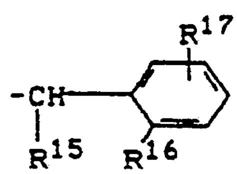 steht, worin

$R^{15}$ für Wasserstoff steht
$R^{16}$ für Fluor, Chlor, Brom, Methyl, Methoxy, Ethoxy, Difluormethoxy, Trifluormethoxy, Methoxy-carbonyl, Ethoxycarbonyl, Methylsulfonyl oder Dimethyllaminosulfonyl steht und

$R^{17}$ für Wasserstoff oder Chlor steht ; worin weiter

$R^1$ für den Rest 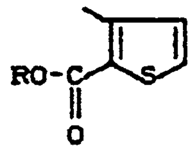 steht, worin

R für $C_1$-$C_2$-Alkyl steht, oder

$R^1$ für den Rest 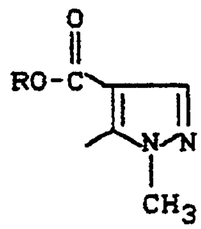 steht, worin

R für $C_1$-$C_2$-Alkyl steht ;
in welcher weiter

$R^2$ für Wasserstoff, Fluor, Chlor, Brom, Methyl, Methoxymethyl, Dimethoxymethyl, Trifluormethyl, Methoxy, Ethoxy, Difluormethoxy, Methylthio, Ethylthio, Amino, Methylamino, Ethylamino, Dimethylamino oder Diethylamino steht,
X für Stickstoff steht,
Y für Stickstoff oder eine —$CR^3$-Gruppierung steht, worin
$R^3$ für Wasserstoff, Fluor, Chlor oder Methyl steht, und
Z für eine —$CR^4$-Gruppierung steht, worin
$R^4$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht,

sowie Salze von Verbindungen der Formel (I), wobei jedoch die Verbindungen N'-(4,6-Dimethyl-pyrimidin-2-yl)-N''-amino-N'''-(2-diethylaminosulfonylphenylsulfonyl)-guanidin, N'-(4,6-Dimethyl-pyrimidin-2-yl)-N''-amino-N'''-(2-methoxycarbonylphenylsulfonyl)-guanidin und N'-(4,6-Dimethylpyrimidin-2-yl)-N''-amino-N'''-(2-methoxycarbonylthiophen-3-yl-sulfonyl)-guanidin ausgenommen sind.

2. Verfahren zur Herstellung von Aminoguanidinoazinen der allgemeinen Formel (I) gemäß Anspruch 1, dadurch gekennzeichnet, daß man Sulfonylverbindungen der allgemeinen Formel (II)

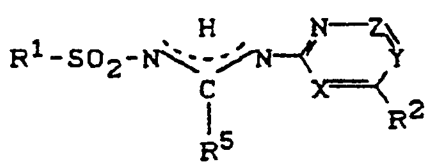 (II)

in welcher
$R^1$, $R^2$, X, Y und Z die in Anspruch 1 angegebenen Bedeutungen haben und
$R^5$ für Halogen oder eine der nachstehend angegebenen Abgangsgruppen

oder —Q-$R^8$ steht, worin

$R^6$ die oben für $R^1$ angegebene Bedeutung hat, aber nicht in jedem Einzelfall mit $R^1$ identisch sein muß,
$R^7$ für $C_1$-$C_4$-Alkyl, $C_3$-$C_4$-Alkenyl oder Benzyl steht,
$R^8$ für gegebenenfalls durch Carboxy, $C_1$-$C_4$-Alkoxy-carbonyl oder $C_1$-$C_4$-Alkoxy substituiertes $C_1$-$C_4$-Alkyl oder für jeweils gegebenenfalls durch Fluor, Chlor, Brom, $C_1$-$C_4$-Alkyl oder $C_1$-$C_4$-Alkoxy substituiertes Benzyl oder Phenyl steht, und
Q für Sauerstoff oder Schwefel steht,

mit Hydrazin oder einem Hydrazin-Wasser- oder Hydrazin- Säure-Addukt, gegebenenfalls in Gegenwart eines Säureakzeptors und gegebenenfalls in Gegenwart eines Verdünnungsmittels umsetzt und gegebenenfalls die so erhaltenen Produkte nach üblichen Methoden in Salze überführt.

3. Herbizide Mittel, gekennzeichnet durch einen Gehalt an mindestens einem Aminoguanidinoazin der Formel (I) gemäß Anspruch 1.

4. Verfahren zur Bekämpfung von Unkräutern, dadurch gekennkeichnet, daß man Aminoguanidinoazine der Formel (I) gemäß Anspruch 1 auf die Unkräuter und/oder ihren Lebensraum einwirken läßt.

5. Verwendung von Aminoguanidinoazinen der Formel (I) gemäß Anspruch 1 zur Bekämpfung von Unkräutern.

6. Verfahren zur Herstellung von herbiziden Mitteln, dadurch gekennzeichnet, daß man Aminoguanidinoazine der Formel (I) gemäß Anspruch 1 mit Streckmitteln und/oder oberflächenaktiven Mitteln vermischt.

**Patentansprüche für folgenden Vertragsstaat : ES**

1. Verfahren zur Herstellung von Aminoguanidinoazinen der allgemeinen Formel (I)

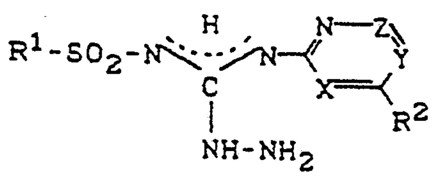

(I)

in welcher

$R^1$ für den Rest 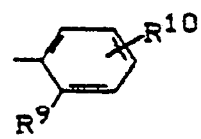 steht worin

$R^9$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxycarbonyl steht und
$R^{10}$ für Wasserstoff oder Chlor steht ; worin weiter

$R^1$ für den Rest 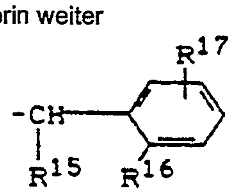 steht, worin

$R^{15}$ für Wasserstoff steht
$R^{16}$ für Fluor, Chlor, Brom, Methyl, Methoxy, Ethoxy, Difluormethoxy, Trifluormethoxy, Methoxy-carbonyl, Ethoxycarbonyl, Methylsulfonyl oder Dimethylaminorulfonyl steht und
$R^{17}$ für Wasserstoff oder Chlor steht ; worin weiter

$R^1$ für den Rest 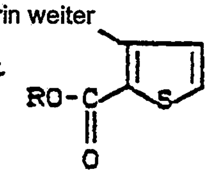 steht, worin

R für $C_1$-$C_2$-Alkyl steht, oder

für den Rest 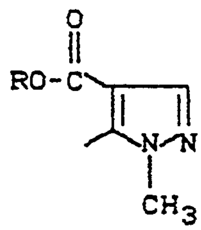 steht, worin

R für $C_1$-$C_2$-Alkyl steht;
in welcher weiter

- $R^2$ für Wasserstoff, Fluor, Chlor, Brom, Methyl, Methoxymethyl, Dimethoxymethyl, Trifluormethyl, Methoxy, Ethoxy, Difluormethoxy, Methylthio, Ethylthio, Amino, Methylamino, Ethylamino, Dimethylamino oder Diethylamino steht,
- X für Stickstoff steht,
- Y für Stickstoff oder eine —$CR^3$-Gruppierung steht, worin
- $R^3$ für Wasserstoff, Fluor, Chlor oder Methyl steht, und
- Z für eine —$CR^4$-Gruppierung steht, worin
- $R^4$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht,

sowie von Salzen von Verbindungen der Formel (I), wobei jedoch die Verbindungen N'-(4,6-Dimethyl-pyrimidin-2-yl)-N''-amino-N'''-(2-diethylaminosulfonyl-phenylsulfonyl)-guanidin, N'-(4,6-Dimethyl-pyrimidin-2-yl)-N''-amino-N'''-(2-methoxycarbonyl-phenylsulfonyl)-guanidin und N'-(4,6-Dimethyl-pyrimidin-2-yl)-N''-amino-N'''-(2-methoxycarbonylthiophen-3-yl-sulfonyl)-guanidin ausgenommen sind, dadurch gekennzeichnet, daß man Sulfonylverbindungen der allgemeinen Formel (II)

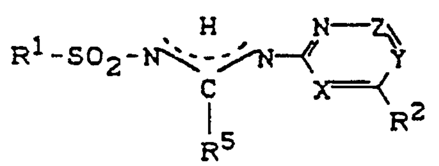 (II)

in welcher
$R^1$, $R^2$, X, Y und Z die oben (bei Formel I) angegebenen Bedeutungen haben und
$R^5$ für Halogen oder eine der nachstehend angegebenen Abgangsgruppen

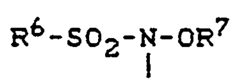

oder —Q-$R^8$ steht, worin

- $R^6$ die oben für $R^1$ angegebene Bedeutung hat, aber nicht in jedem Einzelfall mit $R^1$ identisch sein muß,
- $R^7$ für $C_1$-$C_4$-Alkyl, $C_3$-$C_4$-Alkenyl oder Benzyl steht,
- $R^8$ für gegebenenfalls durch Carboxy, $C_1$-$C_4$-Alkoxy-carbonyl oder $C_1$-$C_4$-Alkoxy substituiertes $C_1$-$C_4$-Alkyl oder für jeweils gegebenenfalls durch Fluor, Chlor, Brom, $C_1$-$C_4$-Alkyl oder $C_1$-$C_4$-Alkoxy substituiertes Benzyl oder Phenyl steht, und
- Q für Sauerstoff oder Schwefel steht,

mit Hydrazin oder einem Hydrazin-Wasser- oder Hydrazin- Säure-Addukt, gegebenenfalls in Gegenwart eines Säureakzeptors und gegebenenfalls in Gegenwart eines Verdünnungsmittels umsetzt und gegebenenfalls die so erhaltenen Produkte nach üblichen Methoden in Salze überführt.

2. Herbizide Mittel, gekennzeichnet durch einen Gehalt an mindestens einem Aminoguanidinoazin der Formel (I) gemäß Anspruch 1.

3. Verfahren zur Bekämpfung von Unkräutern, dadurch gekennzeichnet, daß man Aminoguanidinoazine der Formel (I) gemäß Anspruch 1 auf die Unkräuter und/oder ihren Lebensraum einwirken läßt.

4. Verwendung von Aminoguanidinoazinen der Formel (I) gemäß Anspruch 1 zur Bekämpfung von Unkräutern.

5. Verfahren zur Herstellung von herbiziden Mitteln, dadurch gekennzeichnet, daß man Aminoguanidinoazine der Formel (I) gemäß Anspruch 1 mit Streckmitteln und/oder oberflächenaktiven Mitteln vermischt.

## Claims

**Claims for the following Contracting States : BE, CH, DE, FR, GB, IT, LI, NL, SE**

1. Aminoguanidinoazines of the general formula (I)

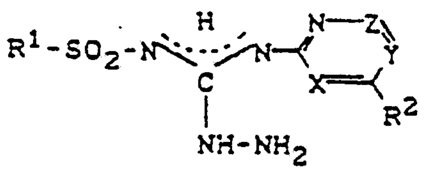

(I)

in which

$R^1$ represents the radical 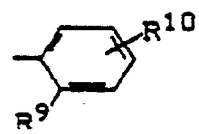 where

$R^9$ represents fluorine, chlorine, bromine, methyl, trifluoromethyl, methoxy, difluoromethoxy, trifluoromethoxy, $C_1$-$C_3$-alkylthio, $C_1$-$C_3$-alkylsulphinyl, $C_1$-$C_3$-alkylsulphonyl, dimethylaminosulphonyl, diethylaminosulphonyl, N-methoxy-N-methylamino-sulphonyl, phenyl, phenoxy or $C_1$-$C_3$-alkoxycarbonyl and

$R^{10}$ represents hydrogen or chlorine ; where furthermore

$R^1$ represents the radical 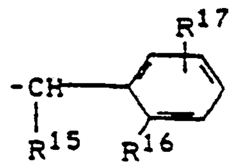 where

$R^{15}$ represents hydrogen

$R^{16}$ represents fluorine, chlorine, bromine, methyl, methoxy, ethyoxy, difluoromethoxy, trifluoromethoxy, methoxycarbonyl, ethoxycarbonyl, methylsulphonyl or dimethylaminosulphonyl and

$R^{17}$ represents hydrogen or chlorine ; where furthermore

$R^1$ represents the radical 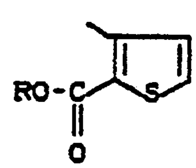 where

R represents $C_1$-$C_2$-alkyl or

$R^1$ represents the radical 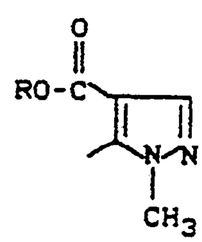 where

R represents $C_1$-$C_2$-alkyl;

in which furthermore

$R^2$ represents hydrogen, fluorine, chlorine, bromine, methyl, methoxyethyl, dimethoxymethyl, trifluoromethyl, methoxy, ethoxy, difluoromethoxy, methylthio, ethylthio, amino, methylamino, ethylamino, dimethylamino or diethylamino,
X represents nitrogen,
Y represents nitrogen or a —$CR^3$ group where
$R^3$ represents hydrogen, fluorine, chlorine or methyl, and
Z represents a —$CR^4$ group where
$R^4$ represents hydrogen, fluorine, chlorine, bromine, methyl, ethyl, trifluoromethyl, methoxy, ethoxy, propoxy, isopropoxy, difluoromethoxy, methylthio, ethylthio, methylamino, ethylamino, dymethylamino or diethylamino, and salts of compounds of the formula (I), with, however, the exception of the compounds N'-(4,6-dimethyl-pyrimidin-2-yl)-N''-amino-N'''-(2-diethyl-aminosulphonyl-phenylsulphonyl)- guanidine, N'-(4,6-dimethyl-pyrimidin-2-yl)-N''-amino-N'''-(2-methoxycarbonyl-phenylsulphonyl)-guanidine and N'-(4,6-dimethyl-pyrimidin-2-yl)-N''-amino-N'''-(2-methoxycarbonyl-thiophen-3-yl-sulphonyl)- guanidine.

2. Process for the preparation of aminoguanidinoazines of the general formula (I) according to Claim 1, characterised in that sulphonyl compounds of the general formula (II)

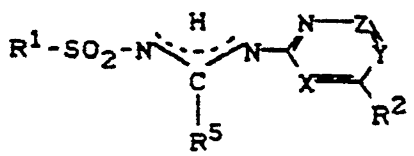

(II)

in which
$R^1$, $R^2$, X, Y and Z have the meanings mentioned in Claim 1 and
$R^5$represents halogen or one of the leaving groups $R^6$-$SO_2$-N-$OR^7$ or —Q-$R^8$ mentioned below where

$R^6$ has the meaning mentioned above for $R^1$ but does not necessarily have to be identical to $R^1$ in each individual case,
$R^7$ represents $C_1$-$C_4$-alkyl, $C_3$-$C_4$-alkenyl or benzyl,
$R^8$ represents $C_1$-$C_4$-alkyl, optionally substituted by carbonyl, $C_1$-$C_4$-alkoxy-carbonyl or $C_1$-$C_4$-alkoxy, or represents benzyl or phenyl, in each case optionally substituted by fluorine, chlorine, bromine, $C_1$-$C_4$-alkyl or $C_1$-$C_4$-alkoxy, and
Q represents oxygen or sulphur,

are reacted with hydrazine or a hydrazine/water or hydrazine/acid adduct, if appropriate in the presence of an acid acceptor and if appropriate in the presence of a diluent, and if appropriate the resulting products are converted to salts by customary methods.

3. Herbicidal agents, characterised in that they contain at least one aminoguanidinoazine of the formula (I) according to Claim 1.

4. Method of combating weeds, characterised in that aminoguanidinoazines of the formula (I) according to Claim 1 are allowed to act on the weeds and/or their environment.

5. Use of aminoguanidinoazines of the formula (I) according to Claim 1 for combating weeds.

6. Process for the preparation of herbicidal agents, characterised in that aminoguanidinoazines of the formula (I) according to Claim 1 are mixed with extenders and/or surface-active agents.

**Claims for the following Contracting State : ES**

1. Process for the preparation of aminoguanidinoazines of the general formula (I)

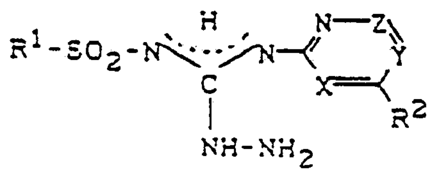

(I)

in which

$R^1$ represents the radical 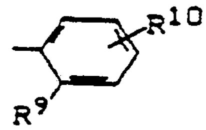 where

$R^9$ represents fluorine, chlorine, bromine, methyl, trifluoromethyl, methoxy, difluoromethoxy, trifluoromethoxy, $C_1$-$C_3$-alkylthio, $C_1$-$C_3$-alkyl-sulphinyl, $C_1$-$C_3$-alkylsulphonyl, dimethylaminosulphonyl, diethylaminosulphonyl, N-methoxy-N-methylamino-sulphonyl, phenyl, phenoxy or $C_1$-$C_3$-alkoxycarbonyl and

$R^{10}$ represents hydrogen or chlorine ; where furthermore

$R^1$ represents the radical 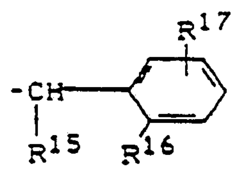 where

$R^{15}$ represents hydrogen

$R^{16}$ represents fluorine, chlorine, bromine, methyl, methoxy, ethoxy, difluoromethoxy, trifluoromethoxy, methoxycarbonyl, ethoxycarbonyl, methylsulphonyl or dimethylaminosulphonyl and

$R^{17}$ represents hydrogen or chlorine ; where furthermore

$R^1$ represents the radical 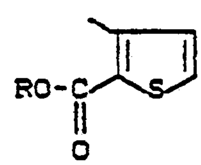 where

R represents $C_1$-$C_2$-alkyl or

$R^1$ represents the radical 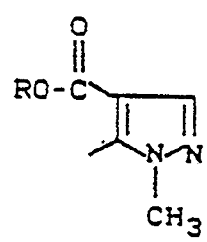 where

R represents $C_1$-$C_2$-alkyl;

in which furthermore

$R^2$ represents hydrogen, fluorine, chlorine, bromine, methyl, methoxymethyl, dimethoxymethyl, trifluoromethyl, methoxy, ethoxy, difluoromethoxy, methylthio, ethylthio, amino, methylamino, ethylamino, dimethylamino or diethylamino,

X represent nitrogen

Y represents nitrogen or a —$CR^3$ group where

$R^3$ represents hydrogen, fluorine, chlorine or methyl, and represents a —$CR^4$ group where

$R^4$ represents hydrogen, fluorine, chlorine, bromine, methyl, ethyl, trifluoromethyl, methoxy, ethoxy, propoxy, isopropoxy, difluoromethoxy, methylthio, ethylthio, methylamino, ethylamino, dimethylamino or diethylamino,

and salts of compounds of the formula (I), with, however, the exception of the compounds N'-(4,6-dimethyl-pyrimidin-2-yl)-N''-amino-N'''-(2-diethyl-aminosulphonyl-phenylsulphonyl)- guanidine, N'-(4,6-dimethyl-pyrimidin-2-yl)-N''-amino-N'''-(2-methoxycarbonyl-phenylsulphonyl)-guanidine and N'-(4,6-dimethyl-pyridin-2-yl)-N''-amino-N'''-(2-methoxycarbonyl-thiophen-3-yl-sulphonyl)- guanidine, characterised in that sulphonyl compounds of the general formula (II)

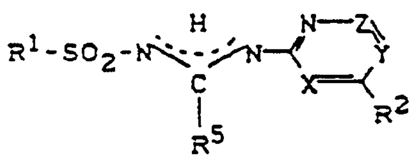 (II)

in which

$R^1$, $R^2$, X, Y and Z have the meanings mentioned above (for formula I) and

$R^5$represents halogen or one of the leaving groups $R^6$-$SO_2$-N-$OR^7$ or -Q-$R^8$ mentioned below where

- $R^6$ has the meaning mentioned above for $R^1$ but does not necessarily have to be identical to $R^1$ in each individual case,
- $R^7$ represents $C_1$-$C_4$-alkyl, $C_3$-$C_4$-alkenyl or benzyl,
- $R^8$ represents $C_1$-$C_4$-alkyl, optionally substituted by carboxyl, $C_1$-$C_4$-alkoxy-carbonyl or $C_1$-$C_4$-alkoxy, or represents benzyl or phenyl, in each case optionally substituted by fluorine, chlorine, bromine, $C_1$-$C_4$-alkyl or $C_1$-$C_4$-alkoxy, and
- Q represents oxygen or sulphur,

are reacted with hydrazine or a hydrazine/water or hydrazine/acid adduct, if appropriate in the presence of an acid acceptor and if appropriate in the presence of a diluent, and if appropriate the resulting products are converted to salts by customary methods.

2. Herbicidal agents, characterised in that they contain at least one aminoguanidinoazine of the formula (I) according to Claim 1.

3. Method of combating weeds, characterised in that aminoguanidinoazines of the formula (I) according to Claim 1 are allowed to act on the weeds and/or their environment.

4. Use of aminoguanidinoazines of the formula (I) according to Claim 1 for combating weeds.

5. Process for the preparation of herbicidal agents, characterised in that aminoguanidinoazines of the formula (I) according to Claim 1 are mixed with extenders and/or surface-active agents.

## Revendications

**Revendications pour les Etats contractants suivants : BE, CH, DE, FR, GB, IT, LI, NL, et SE.**

1. Aminoguanidinoazines de formule générale (I)

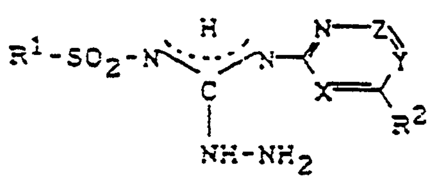 (I)

dans laquelle

$R^1$ représente le groupe 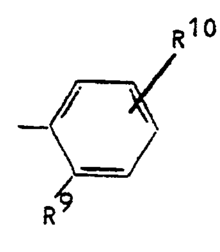 dans lequel

$R^9$ représente le fluor, le chlore, le brome, un groupe méthyle, trifluorométhyle, méthoxy, difluorométhoxy, tri- fluorométhoxy, alkylthio en $C_1$-$C_3$, alkylsulfinyle en $C_1$-$C_3$, alkylsulfonyle en $C_1$-$C_3$, diméthylamino-

sulfonyle, diéthylaminosulfonyle, N-méthoxy-N-méthylaminosulfonyle, phényle, phénoxy ou (alcoxy en $C_1$-$C_3$)-carbonyle, et

$R^{10}$ représente l'hydrogène ou le chlore ; ou bien

$R^1$ représente le groupe 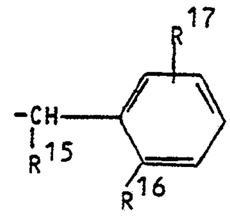 dans lequel

$R^{15}$ représente l'hydrogène,

$R^{16}$ représente le fluor, le chlore, le brome, un groupe méthyle, méthoxy, éthoxy, difluorométhoxy, trifluorométhoxy, méthoxycarbonyle, éthoxycarbonyle, méthylsulfonyle ou diméthylaminosulfonyle, et

$R^{17}$ représente l'hydrogène ou le chlore ; ou bien

$R^1$ représente le groupe 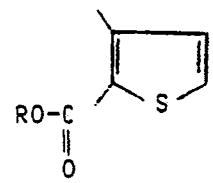 dans lequel

R représente un groupe alkyle en $C_1$-$C_2$, ou bien

$R^1$ représente le groupe 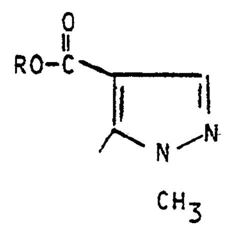 dans lequel

R représente un groupe alkyle en $C_1$-$C_2$ ; et

$R^2$ représente l'hydrogène, le fluor, le chlore, le brome, un groupe méthyle, méthoxyméthyle, diméthoxyméthyle, trifluorométhyle, méthoxy, éthoxy, difluorométhoxy, méthylthio, éthylthio, amino, méthylamino, éthylamino, diméthylamino ou diéthylamino,

X représente l'azote,

Y représente l'azote ou un groupe —$CR^3$— dans lequel

$R^3$ représente l'hydrogène, le fluor, le chlore ou un groupe méthyle, et

Z représente un groupe —$CR^4$—dans lequel

$R^4$ représente l'hydrogène, le fluor, le chlore, le brome, un groupe méthyle, éthyle, trifluorométhyle, méthoxy, éthoxy, propoxy, isopropoxy, difluorométhoxy, méthylthio, éthylthio, méthylamino, éthylamino, diméthylamino ou diéthylamino,

et les sels des composés de formule (I), à l'exclusion des composés : N'-(4,6-diméthylpyrimidine-2-yl)-N''-amino-N'''-(2-diéthylaminosulfonyl-phénylsulfonyl)-guanidine, N'-(4,6-diméthylpyrimidine-2-yl)-N''-amino-'''-(2-méthoxycarbonyl-phénylsulfonyl)- guanidine et N'-(4,6-diméthylpyrimidine-2-yl)-N''-amino-N'''-(2-méthoxy-carbonyl-thiophène-3-yl-sulfonyl)- guanidine.

2. Procédé de préparation des aminoguanidinoazines de formule générale (I) de la revendication 1, caractérisé en ce que l'on fait réagir des dérivés sulfonylés de formule générale (II)

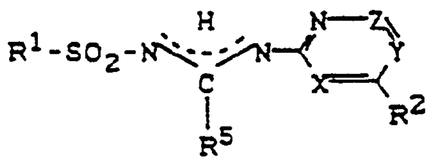

(II)

dans laquelle

$R^1$, $R^2$, X, Y et Z ont les significations indiquées dans la revendications 1, et

$R^5$représente un halogène ou l'un des groupes éliminables

$$R^6-SO_2-\underset{|}{N}-OR^7 \quad \text{ou} \quad -Q-R^8$$

définis ci-après, dans lesquels

- $R^6$ a les significations indiquées ci-dessus pour $R^1$, mais doit être différent dans chaque cas de $R^1$,
- $R^7$ représente un groupe alkyle en $C_1$-$C_4$, alcényle en $C_3$-$C_4$ ou benzyle,
- $R^8$ représente un groupe alkyle en $C_1$-$C_4$ éventuellement substitué par des groupes carboxy, (alcoxy en $C_1$-$C_4$)-carbonyle ou alcoxy en $C_1$-$C_4$, ou un groupe benzyle ou phényle éventuellement substitué par le fluor, le chlore, le brome, un groupe alkyle en $C_1$-$C_4$ ou alcoxy en $C_1$-$C_4$, et
- Q représente l'oxygéne ou le soufre,

avec l'hydrazine ou un adduct hydrazine-eau ou hydrazine-acide, éventuellement en présence d'un accepteur d'acide et éventuellement en présence d'un diluant après quoi, le cas échéant, on convertit les produits obtenus en sels par des modes opératoires usuels.

3. Produits herbicides, caractérisés en ce qu'ils contiennent au moins une aminoguanidinoazine de formule (I) de la revendication 1.

4. Procédé pour la lutte contre les végétaux adventices, caractérisé en ce que l'on fait agir des aminoguanidinoazines de formule (I) de la revendication 1 sur les végétaux adventices et/ou leur habitat.

5. Utilisation des aminoguanidinoazines de formule (I) de la revendication 1 pour la lutte contre les végétaux adventices.

6. Procédé de préparation de produits herbicides, caractérisé en ce que l'on mélange des aminoguanidinoazines de formule (I) de la revendication 1 avec des diluants et/ou des agents tensioactifs.

**Revendication pour l'Etat contractant suivant : ES**

1. Procédé de préparation des aminoguanidinoazines de formule générale (I)

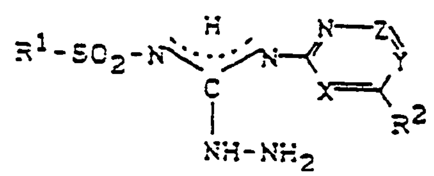

(I)

dans laquelle

$R^1$ représente le groupe 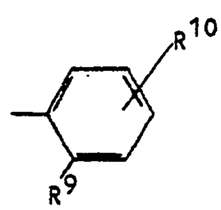 dans lequel

- $R^9$ représente le fluor, le chlore, le brome, un groupe méthyle, trifluorométhyle, méthoxy, difluorométhoxy, trifluorométhoxy, alkylthio en $C_1$-$C_3$, alkylsulfinyle en $C_1$-$C_3$, alkylsulfonyle en $C_1$-$C_3$, diméthylaminosulfonyle, diéthylaminosulfonyle, N-méthoxy-N-méthylaminosulfonyle, phényle, phénoxy ou (alkyle en $C_1$-$C_3$)-carbonyte, et

$R^{10}$ représente l'hydrogène ou le chlore ; ou bien

$R^1$ représente le groupe 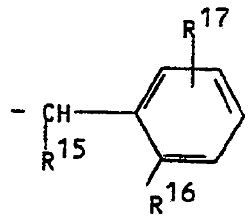 dans lequel

$R^{15}$ représente l'hydrogène,

$R^{16}$ représente le fluor, le chlore, le brome, un groupe méthyle, méthoxy, éthoxy, difluorométhoxy, trifluorométhoxy, méthoxycarbonyle, éthoxycarbonyle, méthylsulfonyle ou diméthylaminosulfonyle, et

$R^{17}$ représente l'hydrogène ou le chlore ; ou bien

$R^1$ représente le groupe 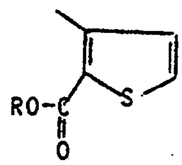 dans lequel

R représente un groupe alkyle en $C_1$-$C_2$, ou bien

$R^1$ représente le groupe 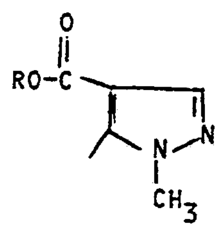 dans lequel

R représente un groupe alkyle en $C_1$-$C_2$, et

$R^2$ représente l'hydrogène, le fluor, le chlore, le brome, un groupe méthyle, méthoxyméthyle, diméthoxyméthyle, trifluorométhyle, méthoxy, éthoxy, difluorométhoxy, méthylthio, éthylthio, amino, méthylamino, éthylamino, diméthylamino ou diéthylamino,

X représente l'azote,

Y représente l'azote ou un groupe —$CR^3$— dans lequel

$R^3$ représente l'hydrogène, le fluor, le chlore ou un groupe méthyle, et

Z représente un groupe —$CR^4$— dans lequel

$R^4$ représente l'hydrogène, le fluor, le chlore, le brome, un groupe méthyle, éthyle, trifluorométhyle, méthoxy, éthoxy, propoxy, isopropoxy, difluorométhoxy, méthylthio, éthylthio, méthylamino, éthylamino, diméthylamino ou diéthylamino,

et des sels des composés de formule (I), les composés suivants étant exclus : N'-(4,6-diméthylpyrimidine-2-yl)-N''-amino-N'''-(2-diéthylaminosulfonyl-phénylsulfonyl)- guanidine, N'-(4,6-diméthyl-pyrimidine-2-yl)-N''-amino-N'''-(2-méthoxycarbonyl-phénylsulfonyl)-guanidine, et N'-(4,6-diméthylpyrimidine-2-yl)-N''-amino-N'''-(2-méthoxycarbonylthiophène-3-yl-sulfonyl)- guanidine, caractérisé en ce que l'on fait réagir des dérivés sulfonylés de formule générale (II)

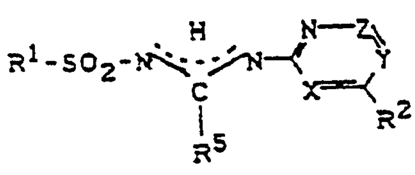 (II)

dans laquelle

$R^1$, $R^2$, X, Y et Z ont les significations indiquées ci-dessus en référence a la formule (I), et
$R^5$représente un halogène ou l'un des groupes éliminables définis ci-après :

$$R^6-SO_2-\underset{|}{N}-OR^7 \quad \text{ou} \quad -Q-R^8$$

dans lesquels

- $R^6$ a les significations indiquées ci-dessus pour $R^1$, mais doit être différent dans chaque cas de celui-ci,
- $R^7$ représente un groupe alkyle en $C_1$-$C_4$, alcényle en $C_3$-$C_4$ ou benzyle,
- $R^8$ représente un groupe alkyle en $C_1$-$C_4$ éventuellement substitué par des groupes carboxy, (alcoxy en $C_1$-$C_4$)-carbonyle ou alcoxy en $C_1$-$C_4$, ou un groupe benzyle ou phényle éventuellement substitué par le fluor, le chlore, le brome, un groupe alkyle en $C_1$-$C_4$ ou alcoxy en $C_1$-$C_4$, et
- Q représente l'oxygène ou le soufre,

avec l'hydrazine ou un adduct d'hydrazine-eau ou hydrazine-acide, éventuellement en présence d'un accepteur d'acide et éventuellement en présence d'un diluant après quoi, le cas échéant, on convertit les produits obtenus en sels par des modes opératoires usuels.

2. Produits herbicides, caractérisés en ce qu'ils contiennent au moins une aminoguanidinoazine de formule (I) de la revendication 1.

3. Procédé pour combattre les végétaux adventices, caractérisé en ce que l'on fait agir des aminoguanidinoazines de formule (I) de la revendication 1 sur les végétaux adventices et/ou leur habitat.

4. Utilisation des aminoguanidinoazines de formule (I) de la revendication 1 pour la lutte contre les végétaux adventices.

5. Procédé de préparation de produits herbicides, caractérisé en ce que l'on mélange des aminoguanidinoazines de formule (I) de la revendication 1 avec des diluants et/ou des agents tensioactifs.